# EUROPEAN PATENT APPLICATION

(11) **EP 4 523 607 A1**
(43) Date of publication of application: **19.03.2025**
(21) Application number: 23197492.4
(22) Date of filing: 14.09.2023
(51) Int. Cl.: A61B 3/00, A61B 3/10

(54) **A MULTI-MODALITY OPHTHALMIC IMAGING SYSTEM AND A METHOD OF IMAGING A PATIENT S EYE**

(71) Applicant: Optos PLC, Fife KY11 8GR (GB)
(72) Inventor: Swan, Derek, Dunfermline, KY11 8GR (GB)
(74) Representative: Hoffmann Eitle

(57) **Abstract**

A multi-modal ophthalmic imaging system (10, 40) for acquiring ophthalmic images of a patient's eye (18) comprising: a first imaging module (14, 42), operable in a first imaging modality, for acquiring a first ophthalmic image (20, 50) of a first portion of the patient's eye (18) and one or more further imaging modules (16, 44, 46), each operable in a different imaging modality. The imaging system further comprises a control module (12) arranged to: detect an anomaly (26, 52) in the first ophthalmic image (20, 50) that is indicative of an ocular disease, determine a region of interest (28, 53, 55) in the patient's eye based on the detected anomaly wherein the region of interest (28, 53, 55) is a region within the patient's eye (18) that is predicted to contain a second anomaly (27, 57) and determine, based on the second anomaly (27, 57), a second imaging modality for imaging the determined region of interest (28, 53, 55) and control a selected second imaging module (16, 44, 46) to acquire a second ophthalmic image of the determined region of interest (28, 53, 55).

## Description

### FIELD

Example aspects herein generally relate to a multi-modality ophthalmic imaging system and to a method of imaging a patient's eye using a multi-modality ophthalmic imaging system.

### BACKGROUND

Multi-modality ophthalmic imaging devices allow clinicians to acquire ophthalmic images of a patient's eye using a variety of different imaging modalities. The imaging modality in which the ophthalmic imaging system is operating is typically selected by a clinician based on the type of pathology the clinician wants to image within the patient's eye. In some instances, the clinician may acquire multiple ophthalmic images of the patient's eye using one or more imaging modalities of the ophthalmic imaging system.

When a clinician is imaging a patient's eye the clinician may first acquire an en-face scanning laser ophthalmoscope (SLO) image of the patient's retina. The clinician may then review the en-face SLO image of the patient's retina to identify one or more regions containing some indication of a pathology that would not be present in a comparable image of a healthy eye. The presence of such region(s) may be indicative of a disease or an otherwise unhealthy state of the subject. Once the clinician has identified the one or more regions that contain an indication of a pathology the clinician may then capture a further ophthalmic image, such as an ophthalmic coherence tomography (OCT) image of the region of the eye using one or more different imaging modalities.

This process of capturing an initial ophthalmic image, identifying regions indicative of disease and subsequently capturing further ophthalmic images of the patient's eye using further imaging modalities can be very time consuming for both the clinician and patient. Furthermore, if the ophthalmic imaging system is being operated by a photographer that is unskilled in diagnosing ophthalmic disease the initial ophthalmic image may have to be sent to be reviewed by an ophthalmologist for analysis to determine if further ophthalmic images are required. This can result in the patient having to be recalled to the clinic multiple times to be imaged multiple times by the photographer based on the feedback from the ophthalmologist.

The process of reviewing an initial ophthalmic image to identify pathologies is a time-consuming process that requires a highly skilled ophthalmologist to review the images. This can significantly reduce the throughput of patients through a clinic and often requires patients to be recalled to clinics for further ophthalmic images to be captured in order to diagnose ocular disease.

### SUMMARY

There is provided, in accordance with a first example aspect a multi-modal ophthalmic imaging system for acquiring ophthalmic images of a patient's eye, the multi-modal ophthalmic imaging system comprising: a first imaging module, operable in a first imaging modality, for acquiring a first ophthalmic image of the patient's eye; one or more further imaging modules, each operable in a respective imaging modality, wherein each imaging modality of the one or more further imaging modules is different to the first imaging modality; and a control module arranged to: detect an anomaly in the first ophthalmic image acquired by the first ophthalmic imaging module; determine a region of interest in the patient's eye based on the detected anomaly in the first ophthalmic image, wherein the determined region of interest is a region within the patient's eye that is predicted to contain a second anomaly; determine, based on the second anomaly, a second imaging modality for imaging the determined region of interest, the second imaging modality being different from the first imaging modality; select, from the one or more further imaging modules, a second imaging module which is operable in the determined second imaging modality; and control the selected second imaging module to acquire a second ophthalmic image of the determined region of interest.

In an example embodiment the anomaly detected in the first ophthalmic image may be indicative of a first stage of a multi-stage or progressive ocular disease and the second anomaly may be indicative of a second stage of the multi-stage progressive ocular disease.

In another example embodiment the first imaging module may comprise a scanning laser ophthalmoscope (SLO), and the first ophthalmic image may be a fundus image of the patient's eye.

In a further example embodiment the control module may be further arranged to determine, based on the second anomaly, one or more image acquisition parameters of the second imaging module for controlling the second imaging module to acquire the second ophthalmic image of the determined region of interest.

In an example embodiment the control module may be communicatively coupled to a database comprising a medical record indicative of a medical history of the patient, and wherein the control module is arranged to retrieve the medical record of the patient from the database and determine the region of interest based on both the detected anomaly and the medical history of the patient.

In an example embodiment the control module may be configured to detect a discontinuity at a boundary of the second ophthalmic image, wherein the discontinuity at the boundary is indicative of cropping of the second anomaly located within the second ophthalmic image. The control module may be arranged, in response to detecting the discontinuity at the boundary of the second image, to increase a size of the determined region of interest to generate an enlarged region of interest; and control the second imaging module to acquire an ophthalmic image of the enlarged region of interest.

In another example embodiment the control module may be arranged to: determine a second region of interest based on the detected anomaly wherein the second region of interest is a region that contains the detected anomaly; determine, based on the detected anomaly, a third imaging modality for imaging the determined second region of interest, the third imaging modality being different from the first imaging modality; select, from the one or more further imaging modules, a third imaging module which is operable in the determined third imaging modality; and control the selected third imaging module to acquire a third ophthalmic image of the determined second region of interest.

There is provided, in accordance with a second example aspect, a method of imaging a patient's eye, the method comprising: acquiring a first ophthalmic image of the patient's eye using a first imaging module operating in a first imaging modality; detecting an anomaly in the first ophthalmic image acquired by the first ophthalmic imaging module; determining a region of interest in the patient's eye based on the detected anomaly in the first ophthalmic image, wherein the determined region of interest is a region within the patient's eye that is predicted to contain a second anomaly; determining, based on the second anomaly, a second imaging modality for imaging the determined region of interest, the second imaging modality being different from the first imaging modality; and acquiring a second ophthalmic image of the determined region of interest using a second imaging module operating in the determined second imaging modality.

In an example embodiment the anomaly detected in the first ophthalmic image may be indicative of a first stage of a multi-stage or progressive ocular disease and the second anomaly may be indicative of a second stage of the multi-stage or progressive ocular disease.

In one example embodiment the method may comprise determining if the second anomaly is captured within the second ophthalmic image by detecting a discontinuity at a boundary of the second ophthalmic image, wherein detecting the presence of a discontinuity at the boundary may be indicative of cropping of the second anomaly within the second ophthalmic image.

In an example embodiment the method may comprise, in response to detecting the presence of the discontinuity at the boundary of the second ophthalmic image, increasing a size of the determined region of interest to generate an enlarged region of interest and acquiring a further ophthalmic image of the enlarged region of interest.

In a further example embodiment the method may comprise receiving medical records of the patient containing a medical history of the patient, and wherein determining the region of interest may be based on a combination of the detected anomaly in the first ophthalmic image and the received medical history of the patient.

In another example embodiment the method may further comprise determining, based on the second anomaly, one or more image acquisition parameters of the second imaging module and operating the second imaging module in the determined image acquisition parameters to acquire the second ophthalmic image.

In an example embodiment the method may comprise determining a second region of interest based on the detected anomaly, wherein the second region of interest is a region that contains the detected anomaly; determining, based on the detected anomaly, a third imaging modality for imaging the determined second region of interest, the third imaging modality being different from the first imaging modality; selecting, from the one or more further imaging modules, a third imaging module which is operable in the determined third imaging modality; and controlling the selected third imaging module to acquire a third ophthalmic image of the determined second region of interest.

In a third example aspect there is provided a multi-modal ophthalmic imaging system for acquiring ophthalmic images of a patient's eye, the multi-modal ophthalmic imaging system comprising: a first imaging module, operable in a first imaging modality, for acquiring a first ophthalmic image of the patient's eye; one or more further imaging modules, each operable in a respective imaging modality, wherein each imaging modality of the one or more further imaging modules is different to the first imaging modality; and a control module arranged to: detect an anomaly in the first ophthalmic image acquired by the first ophthalmic imaging module, the anomaly being indicative of a first stage of a multi-stage ocular disease; predict a second stage of the multi-stage ocular disease based on the detected anomaly; determine a region of interest in the patient's eye based on the predicted second stage of the multi-stage ocular disease, wherein the determined region of interest is a region within the patient's eye that is predicted to contain a second anomaly indicative of the second stage of the multi-stage ocular disease; determine, based on the second anomaly, a second imaging modality for imaging the determined region of interest, the second imaging modality being different from the first imaging modality; select, from the one or more further imaging modules, a second imaging module which is operable in the determined second imaging modality; and control the selected second imaging module to acquire a second ophthalmic image of the determined region of interest.

In a fourth example aspect there is provided a method of imaging a patient's eye, the method comprising: acquiring a first ophthalmic image of the patient's eye using a first imaging module operating in a first imaging modality; detecting an anomaly in the first ophthalmic image acquired by the first ophthalmic imaging module; predicting a second stage of the multi-stage ocular disease based on the detected anomaly; determining a region of interest in the patient's eye based on the predicted second stage of the multi-stage ocular disease, wherein the determined region of interest is a region within the patient's eye that is predicted to contain a second anomaly indicative of the second stage of the multi-stage ocular disease; determining, based on the second anomaly, a second imaging modality for imaging the determined region of interest, the second imaging modality being different from the first imaging modality; and acquiring a second ophthalmic image of the determined region of interest using a second imaging module operating in the determined second imaging modality.

### BRIEF DESCRIPTION OF THE DRAWINGS

Example embodiments will now be explained in detail, by way of non-limiting example only, with reference to the accompanying figures described below. Like reference numerals appearing in different ones of the figures can denote identical or functionally similar elements, unless indicated otherwise.
Figure 1 is a schematic illustration of a multi-modal ophthalmic imaging system according to a first example embodiment herein.
Figure 2 is a schematic illustration of a fundus image captured by the multi-modal ophthalmic imaging system of Figure 1.
Figure 3 is a flow chart illustrating a method of imaging a patient's eye according to an example embodiment herein.
Figure 4 is a schematic illustration of a multi-modal ophthalmic imaging system according to a second example embodiment herein.
Figure 5 is a schematic illustration of a first ophthalmic image captured by a first imaging module within the multi-modal ophthalmic imaging system of Figure 1 or Figure 4.
Figure 6 is an example hardware implementation of an apparatus that can operate as a control module for a multi-modality ophthalmic imaging system according to an example embodiment herein.

### DETAILED DESCRIPTION

In view of the background provided above, the inventor has devised a multi-modality ophthalmic imaging system having a control module or processor arranged to detect an anomaly indicative of a first stage of a progressive or a multi-stage ocular disease in a first ophthalmic image acquired by a first ophthalmic imaging module operating in a first imaging modality. Upon detection of the anomaly in the first ophthalmic image, the control module is arranged to determine one or more regions of interest within the patient's eye based on the detected anomaly. The one or more determined regions of interest may be regions of the patient's eye that are susceptible to a progression or further stage of the multi-stage ocular disease that the detected anomaly is indicative of. The control module may determine a second imaging modality for acquiring a second ophthalmic image of the determined region of interest based on the detected anomaly. The control module is configured to select a second imaging module that is operable in the determined second imaging modality to acquire a second ophthalmic image of the one or more regions of interest using the second imaging module to acquire further ophthalmic images of regions within the patient's eye that are susceptible to a progression of the multi-stage ocular disease and that are predicted to or expected to contain a second anomaly that is indicative of a progression of the multi-stage ocular disease.

For example, the multi-modality ophthalmic imaging system may be an ophthalmic imaging system having a scanning laser ophthalmoscope (SLO) imaging module and an optical coherence tomography (OCT) imaging module. The SLO imaging module may acquire a fundus image of a patient's eye. The control module may analyse or search the captured fundus image to check for the presence of an anomaly that is indicative of a multi-stage or progressive ocular disease. Upon detection of an anomaly in the fundus image the control module may determine a region of interest in the fundus image that is predicted to contain a second anomaly that is associated with a progression of the multi-stage ocular disease that the detected anomaly is indicative of. The determined region of interest may be a region for further imaging by the second imaging modality. For example, the region of interest may be a region in the periphery of the patient's retina that is predicted to contain a second anomaly that is associated with a progression of the multi-stage ocular disease based on the detection of the anomaly in the fundus image.

The control module is configured to select a second imaging modality for acquiring an ophthalmic image of the determined region of interest based on the detected anomaly and/or based on a type of pathology that is expected to be present as a second anomaly in the determined region of interest based on an expected progressed stage of the multi-stage ocular disease that the detected anomaly is indicative of. For example, if the control module predicts that the second anomaly will be retinal bleeding such that retinal bleeding may be present at the determined region of interest the control module may determine that the OCT imaging module should be operated in an OCT angiography (OCTA) imaging modality to acquire a second ophthalmic image of the determined region of interest. The OCT imaging module is then operated in an OCTA imaging modality to acquire the second ophthalmic image of the determined region of interest.

Advantageously, the multi-modality ophthalmic imaging system allows multiple images of the patient's eye to be acquired in a single imaging session. This can help to capture the images necessary to accurately diagnose a level of progression of a multi-stage ocular disease in a patient's eye in a single visit to the clinician by the patient. Furthermore, the control module can select the second imaging modality for imaging the predicted second anomaly contained within the determined region of interest based on the detected anomaly and/or based on the second anomaly that is expected to be contained within the determined region of interest. This beneficially allows the multi-modality ophthalmic imaging system to be operated to capture high-quality ocular images of anomalies in the patient's eye which assists the ophthalmologist in diagnosing a state of progression of ocular disease in the patient. Furthermore, the control module can vary acquisition parameters of the second imaging module based on the detected anomalies and/or based on the predicted second anomaly associated with a progression of the multi-stage ocular disease that is expected to be present in the determined region of interest to optimise the quality of the second ophthalmic image. Optimising the quality of the second ophthalmic image allows the ophthalmic imaging system to acquire high quality images of the patient's eye such that an ophthalmologist can accurately diagnose and assess the severity of the ocular disease present in the patient's eye without requiring the patient to return to the clinic for further imaging.

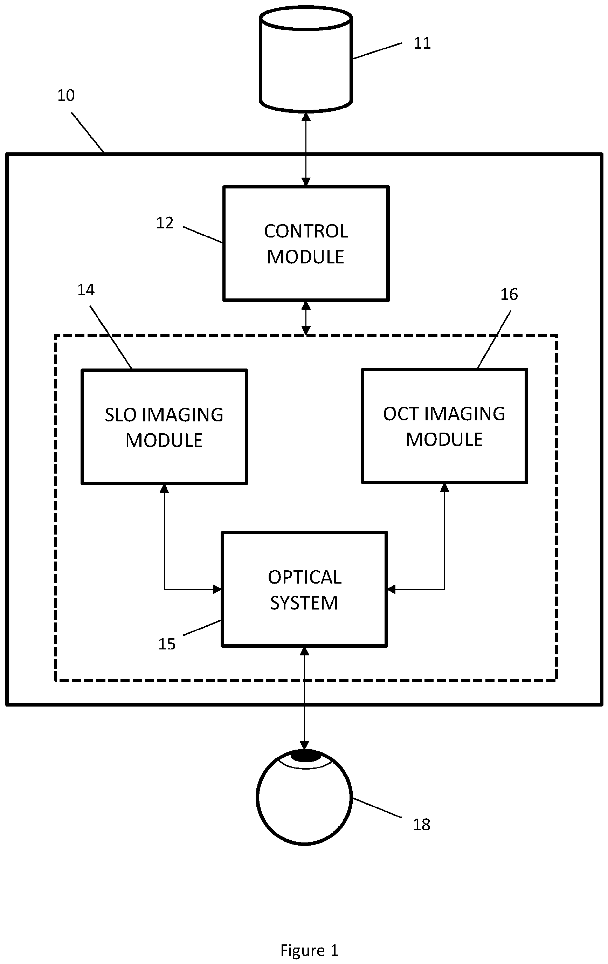

To place example embodiments in a suitable context, reference will now be made to Figure 1, which schematically shows a multi-modality ophthalmic imaging system 10 for imaging a patient's eye 18. The multi-modality ophthalmic imaging system 10 comprises a scanning laser ophthalmoscope (SLO) imaging module 14 and an optical coherence tomography (OCT) imaging module 16 for acquiring SLO and OCT images of the patient's eye 18 respectively. The SLO imaging module 14 is an example of a first imaging module or system and the OCT imaging module 16 is an example of a second imaging module or system. The SLO imaging module 14 and OCT imaging module 16 may each be operable in a plurality of different imaging modalities.

The ophthalmic imaging system 10 may, as in the present example embodiment, further comprise an optical system 15 which is optically coupled to the SLO and OCT imaging modules 14, 16 and arranged to convey light from the SLO and OCT imaging modules 14, 16 to and from the patient's eye 18. The optical system 15 may comprise focusing optics and a scanning system (not shown) for scanning a light beam emitted by the SLO and/or OCT imaging modules 14, 16 across a target imaging region of the patient's eye 18. For example, the scanning system may comprise one or more scanning mirrors or galvanometers for scanning the light beam emitted by either of the imaging modules 14, 16 across a target imaging area of the eye 18 such as across a region of interest in the patient's retina. The SLO and OCT imaging modules 14, 16 may comprise one or more photodetectors (not shown) for detecting light reflected by the patient's eye 18, and a signal processing device (not shown) arranged to generate ophthalmic images based on an output of the photodetector(s). The optical system 15 is arranged to convey light reflected by the patient's eye 18 to the photodetectors located within the SLO and OCT imaging modules 14, 16 such that the signal processing device can generate ophthalmic images of the patient's eye 18 based on light reflected or scattered by the eye 18.

The ophthalmic imaging system 10 further comprises a processor or control module 12 coupled to the SLO imaging module 14, the OCT imaging module 16 and the optical system 15. The control module 12 is configured to control the SLO imaging module 14, the OCT imaging module 16 and the optical system 15. The processor or control module 12 is a component of the multi-modality ophthalmic imaging system 10 and is coupled to the SLO imaging module 14, the OCT imaging module 16 and the optical system 15, for controlling those components and for exchanging data and information therebetween.

The control module 12 is arranged to select an operating modality of the multi-modality ophthalmic imaging system 10 and operate one or more of the SLO and OCT imaging modules 14, 16 to acquire ophthalmic images of the patient's eye 18 in dependence on the selected operating modality. Furthermore, the control module 12 can control the optical system 15 to select a target imaging area of the patient's eye 18 to be imaged such that the scanning system within the optical system 15 can scan the light beam emitted by either of the SLO and OCT imaging modules 14, 16 over a target imaging area within the patient's eye 18 to acquire one or more ophthalmic images of the selected imaging area.

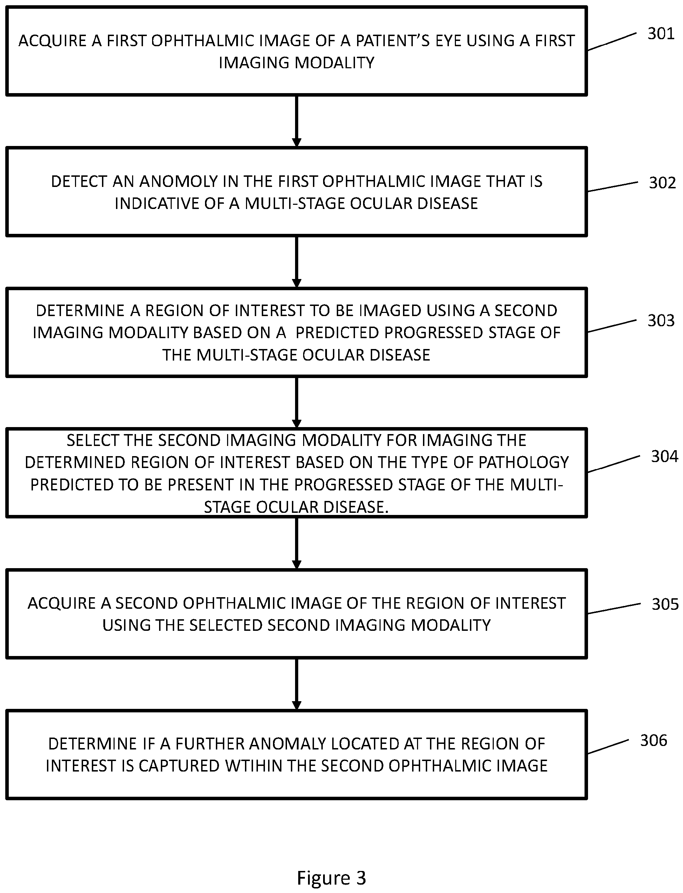

The multi-modality ophthalmic imaging system 10 may further be communicatively coupled to a database 11. The database 11 may comprise medical records of a patient being imaged by the multi-modality ophthalmic imaging system 10. The medical records of the patient include a medical history of the patient. The control module 12 can access the medical records of the patient and retrieve the medical history of the patient. Advantageously, the control module 12 may review the retrieved medical history for conditions that may manifest in ocular disease as is discussed in further detail below with reference to Figure 3.

The multi-modality ophthalmic imaging system 10 is arranged to acquire a first ophthalmic image of the patient's eye 18 using a first imaging module operable in a first imaging modality. With reference to Figure 1, the first ophthalmic imaging module may be the SLO imaging module 14 operating in a fundus imaging modality. The SLO imaging module 14 may acquire a fundus image of the patient's eye 18. The fundus image acquired by the SLO imaging module 14 is an example of a first ophthalmic image acquired by a first ophthalmic imaging system.

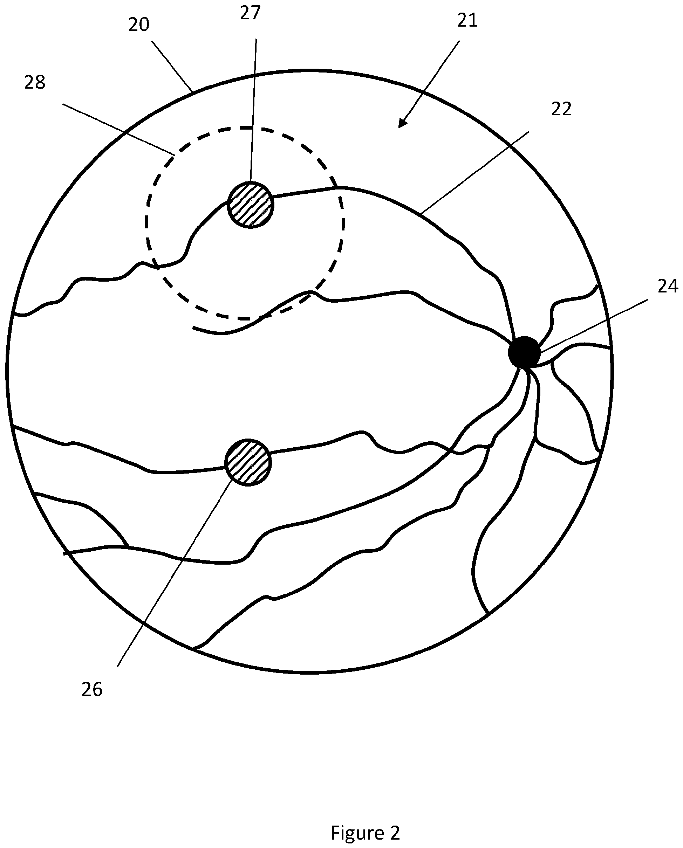

Turning now to Figure 2 there is shown a schematic fundus image 20 of the patient's eye 18. The fundus image 20 may be acquired by the SLO imaging module 14 of the multi-modality ophthalmic imaging system 10. The fundus image 20 comprises retinal vasculature 22 extending across the patient's retina 21 from the optic disc 24 towards the retinal periphery. The fundus image 20 comprises an anomaly 26 located on the patient's retina 21 that is indicative of a first stage in a multi-stage or progressive ocular disease. The anomaly 26 is a feature in the fundus image 20 that is indicative of a multi-stage or progressive ocular disease within the eye 18 such as diabetic retinopathy or a tumour. The anomaly 26 may be one or more of: lesions, a retinal hole, a retinal detachment, fluid build-up, a tumour, discoloration of the retina 21, a shadow on the retina 21, bleeding of retinal vasculature 22, microaneurysms in the vasculature 22 or any other feature that is indicative of a multi-stage ocular disease that presents as an anomaly 26 on the surface or sub-surface of the patient's retina 21.

The fundus image 20 is an example of a first ophthalmic image captured by a first imaging module which in this example embodiment may be the SLO imaging module 14. Upon acquiring the fundus image 20, the SLO imaging module 14 may transmit the fundus image 20 to the control module 12. The control module 12 is configured to analyse the received fundus image 20 to detect the presence of one or more anomalies 26 indicative of a first stage of a multi-stage ocular disease within the fundus image 20. The control module 12 may use image processing techniques or a machine learning algorithm that has been trained on ocular image data comprising images of healthy and diseased eyes, to detect anomalies 26 indicative of multi-stage ocular disease within an ophthalmic image to detect the presence of anomalies within the fundus image 20. Such techniques for searching for an anomaly in an ophthalmic image are described in further detail in WO 2020/127233 A1, the content of which is hereby incorporated by reference in its entirety.

If the control module 12 detects the presence of one or more anomalies 26 within the fundus image 20 that are indicative of a stage within a multi-stage or progressive ocular disease then the control module 12 is configured to determine a region of interest 28 based on the detection of the anomaly 26 in the fundus image 20 wherein the determined region of interest is predicted or expected to contain a second anomaly 27 that is indicative of a further stage or a progression of the multi-stage ocular disease. The second anomaly 27 may be an anomaly that is not visible in the first ophthalmic image. For example, the second anomaly 27 may be a sub-surface feature or a feature outside the field of view of the first ophthalmic image 20.

If the anomaly 26 is indicative of bleeding into the retina then the anomaly 26 may be indicative of pre-proliferative retinopathy in the case of diabetic retinopathy. In this instance, the next stage in diabetic retinopathy is proliferative retinopathy which can result in retinal detachment. As such, the region of interest 28 may be a region within the eye 18 that is susceptible to retinal detachments, for example, a peripheral region of the retina and the second anomaly 27 may be predicted to be a retinal detachment.

The determined region of interest 28 is an area of the patient's eye 18 that is predicted to contain a second anomaly 27 and that is to be imaged by an imaging module operating in a different imaging modality to the imaging modality used to acquire the first ophthalmic image, in this example the fundus image 20. The determined region of interest 28 may be a region that encircles the detected anomaly 26 and the second anomaly 27 may be predicted to be an enlargement of the detected anomaly 26 or, as shown in Figure 2, the region of interest 28 may be a region that is remote from the detected anomaly 26 that is determined to be a region of interest in the patient's eye that is susceptible of disease associated with the detected anomaly 26 and that is predicted to contain the second anomaly 27. For example, if the anomaly 26 is indicative of a pathology that starts in a central region of the retina 21 and progresses to the periphery of the retina 21 the determined region of interest 28 may be located at the periphery of the retina 21. The size, position and shape of the region of interest 28 may be determined by the control module 12 based on characteristics of the detected anomaly 26.

The detected anomaly 26 may be an area of swelling in a blood vessel on the patient's retina 21 that is indicative of early stages of diabetic retinopathy. For example, the swelling may be a micro-aneurysm indicative of mild non-proliferative diabetic retinopathy. In an example where the anomaly 26 is indicative of early stages of diabetic retinopathy the region of interest 28 may be determined to be an area that surrounds the detected micro-aneurysms and an OCTA (OCT-angiography) imaging modality may be used to acquire further ophthalmic images of the determined region of interest 28 to establish if there is a second anomaly 27 present that is associated with bleeding into the retina indicative of pre-proliferative retinopathy.

The control module 12 is configured to determine a second imaging modality for acquiring a second ophthalmic image of the determined region of interest 28 based on the detected anomaly 26 in the first ophthalmic image and/or based on the predicted second anomaly 27. The second imaging modality for imaging the determined region of interest 28 may be determined based on the type of pathology that is expected to be present in the determined region of interest 28 as the predicted second anomaly 27. The type of pathology that the second anomaly 27 is expected to be present in the determined region of interest 28 is a pathology that is associated with a progression of the multi-stage ocular disease that the detected anomaly 26 is indicative of.

The control module 12 may determine the multi-stage ocular disease that the anomaly 26 is indicative of based on one or more characteristics of the anomaly 26 detected in the first ophthalmic image 20. The characteristic of the anomaly 26 may be any one or more of a: size, shape, position, colour, number of occurrences of the anomaly or any other feature indicative of multi-stage ocular disease. Based on the characterisation of the anomaly 26 the control module 12 is arranged to predict the location within the eye and also predict the type of pathology that is likely to be present as a second anomaly 27 at the predicted location and then determine a second imaging modality for acquiring a second ophthalmic image of the determined region of interest 28 based on the predicted second anomaly 27.

The control module 12 may use a machine learning algorithm or image processing technique to determine a characteristic of the detected anomaly 26 to predict the type of multi-stage ocular disease the detected anomaly 26 is indicative of. The size, shape and position of the region of interest 28 is determined based on the type of multi-stage ocular disease that the detected anomaly 26 is indicative of and/or based on the second anomaly 27 that is predicted to be present in the region of interest 28.

Based on the predicted second anomaly 27 the control module 12 determines a second imaging modality to be used for imaging the determined region of interest 28 and then selects a second imaging module operable in the second imaging mode to acquire the second ophthalmic image. For example, the control module 12 may determine that an OCT image of the region of interest 28 should be acquired to obtain cross-sectional image data of the predicted second anomaly 27. In this instance, the control module 12 may select the OCT imaging module 16 operating in an OCT imaging mode to acquire the second ophthalmic image. Alternatively, the control module 12 may determine that an autofluorescence modality is required to obtain a second ophthalmic image of the predicted second anomaly 27. In this instance, the control module 12 may select the SLO imaging module 12 operating in an autofluorescence imaging modality to acquire the second ophthalmic image of the region of interest 28.

The control module 12 is configured to operate the selected second imaging module to acquire the second ophthalmic image of the determined region of interest 28. Imaging the determined region of interest 28 that is predicted to contain the second anomaly 27 associated with a progression of a multi-stage ocular disease that is synonymous with the detected anomaly 26 using an imaging modality that is selected based on a characteristic of the detected anomaly 26 and/or based on the predicted second anomaly 27 associated with a progression of the multi-stage ocular disease in the first ophthalmic image allows high quality images of the anomaly 26 and/or the second anomaly 27 associated with the progression of the multi-stage ocular disease to be acquired with minimal input from the operator of the ophthalmic imaging system 10. This beneficially allows a relatively unskilled operator to photograph a patient using the multi-modality ophthalmic imaging system 10 whilst still capturing high quality ophthalmic images of a patient's eye 18 that can be transferred to an ophthalmologist for diagnosis and assessment of a multi-stage ocular disease.

The control module 12 is further configured to determine acquisition characteristics or parameters of the second imaging module based on characteristics of the detected anomaly 26 and/or based on the type of pathology that is predicted to be present in the determined region of interest 28 in the form of the second anomaly 27. For example, the control module 12 may determine acquisition parameters of the second imaging module which can include determining one or more of: the scan field, imaging depth, imaging wavelength, scan pattern, scan speed and scan density for imaging the region of interest 28. The acquisition characteristics of the second imaging module may be selected based on the type of pathology associated with the predicted progressed stage of the multi-stage ocular disease. For example, if the control module 12 predicts that the second anomaly 27 associated with the predicted progressed stage of the multi-stage ocular disease is sub-retinal fluid then the acquisition characteristics of the OCT imaging module 16 (or second imaging module) may be set to acquire a large field, large depth OCT image of the region of interest 28 so as to capture the entire area that is predicted to contain the sub-retinal fluid. Furthermore, the position and shape of the determined region of interest 28 may affect the acquisition characteristics of the second imaging module. For example, the scan pattern, scan field, imaging depth and imaging wavelength may be determined based on the position and shape of the determined region of interest 28.

The ophthalmic imaging system 10 may comprise a display (not shown) arranged to display images acquired by the ophthalmic imaging system 10 to a user of the ophthalmic imaging system 10. The control module 12 may be coupled to the display such that the display can show the first ophthalmic image, fundus image 20, to a user of the ophthalmic imaging system 10. Furthermore, the display may overlay the determined region of interest 28 on the fundus image 20 such that a user of the ophthalmic imaging system 10 can view the position of the determined region of interest 28 relative to the patient's retina 21 on the first ophthalmic image. In an example embodiment the clinician can reposition and/or resize the determined region of interest 28.

The ophthalmic imaging system 10 of Figure 1 is communicatively coupled to a database 11. As shown in Figure 1, the control module 12 may be coupled to the database 11. The database 11 may form part of the ophthalmic imaging system 10 or the database may be a remote database 11. For example, the database 11 may be stored on the cloud and accessed wirelessly by the control module 12.

The database 11 is a database containing medical information relating to the patient being imaged by the ophthalmic imaging system 10. For example, the database 11 may comprise digital medical records relating to the medical history of the patient. The control module 12 is configured to access the database 11 to retrieve the medical records of the patient being imaged by the ophthalmic imaging system 10. For example, the control module 12 may access the database 11 to retrieve the patient's medical records to review the records for medical conditions that may manifest in ocular disease, and in particular, but not exclusively, in multi-stage ocular disease. For example, the control module 12 may review the patient's medical history to check for previous instances of ocular disease or for conditions such as diabetes or the patient's age that may make the patient at risk of a multi-stage ocular disease such as diabetic retinopathy or age-related macular degeneration.

Upon receipt of the first ophthalmic image, for example the fundus image 20, the control module 12 is arranged analyse the first ophthalmic image for anomalies 26 within the image. The control module 12 may modify the analysis of the first ophthalmic image based on the patient's medical records. For example, if the patient is diabetic the control module 12 may search the first ophthalmic image for anomalies 26 indicative of diabetic retinopathy such as micro-aneurysms, bleeding into the retina, a retinal detachment and blocked or leaking blood vessels in the macula.

Furthermore, if the control module 12 detects the presence of an anomaly 26 indicative of ocular disease the control module 12 may determine the region of interest 28 that is predicted to contain the second anomaly 27 and/or select the second imaging modality for imaging the region of interest 28 based on a combination of the patient's medical history and the characterisation of the anomaly 26. For example, if the patient's medical history shows that the patient is diabetic and an anomaly 26 indicative of neovascularisation is detected, the control module 12 may determine a region of interest 28 and select the second imaging modality to investigate the progression of diabetic retinopathy. In this example, the control module 12 may determine an OCT angiography (OCTA) imaging modality to assess the severity of the neovascularisation (second imaging modality 27) within the retina should be used and the control module 12 may then select the OCT imaging module 16 operating in an OCTA modality as the second imaging module to acquire the second ophthalmic image. If medical records of the patient are available the control module 12 may determine the second imaging modality based on a combination of one or more of: a characteristic of the detected anomaly 26, a type of pathology predicted to be present in the determined region of interest and the medical history of the patient.

Figure 3 is a flow chart illustrating a method of imaging a patient's eye 18. The method may be performed using the multi-modality ophthalmic imaging system 10 of Figure 1.

In Step 301 the multi-modality ophthalmic imaging system 10 is operated in a first imaging modality to acquire a first ophthalmic image of the patient's eye 18. Acquiring the first ophthalmic image of the patient's eye 18 may comprise operating a first imaging module operable in the first imaging modality to acquire a first image of the patient's eye 18. For example, the SLO imaging module 14 of the ophthalmic imaging system 10 may be operated in a fundus imaging modality to acquire a fundus image 20 of the patient's retina 21.

Next, in Step 302, the first ophthalmic image of the patient's eye 18 acquired in Step 301 is analysed or searched for the presence of anomalies indicative of a multi-stage or progressive ocular disease. Step 302 comprises detecting an anomaly 26 in the first ophthalmic image captured by the first ophthalmic imaging module, wherein the detected anomaly 26 is an anomaly that is indicative of a multi-stage ocular disease. Detecting the presence of an anomaly 26 that is indicative of ocular disease in the first ophthalmic image may comprise using image processing techniques or machine learning algorithms to detect the presence of the anomaly 26 in the first ophthalmic image. For example, an artificial intelligence ophthalmic image analysis model may be used to detect the presence of an anomaly 26 in the first ophthalmic image. Detecting the anomaly 26 may comprise one or more of detecting a discoloration on the retina, a lesion, a high or low rate of vasculature, a hole in the retina or any other anomaly indicative of ocular disease.

Detecting the anomaly 26 in the first ophthalmic image may further comprise determining a stage of the multi-stage ocular disease that the anomaly 26 is indicative of. For example, if the anomaly 26 is a micro-aneurysm on the vasculature of the patient's retina then the method may further comprise determining that the anomaly 26 is indicative of background retinopathy which is the first stage of diabetic retinopathy.

Upon detection of an anomaly 26 in the first ophthalmic image in Step 302 the method comprises determining a region of interest 28 within the patient's eye 18 that is predicted to contain a second anomaly 27 in Step 303. Determining the region of interest 28 may comprise determining a region of interest 28 within the patient's eye 18 to be imaged using a second imaging modality based on the detection of an anomaly 26 in the first ophthalmic image. Determining the region of interest 28 may comprise predicting a location or region within the patient's eye 18 that is susceptible to a progressed stage of the multi-stage ocular disease and within which a second anomaly 27 is predicted to manifest. The determined region of interest 28 may be a region within the patient's eye 18 that is susceptible to a second or progressed stage of the multi-stage ocular disease that the detected anomaly 26 is indicative of and that is predicted or expected to contain a second anomaly 27.

In the example described above where the anomaly 26 is indicative of background retinopathy (Stage 1 in diabetic retinopathy) in a patient suffering from diabetic retinopathy, the region of interest 28 may be a region that encircles the detected anomaly 26. The region of interest 28 in this example may be a region of the retina 21 that is susceptible to bleeding caused by pre-proliferative retinopathy (Stage 2 of diabetic retinopathy) and the predicted second anomaly 27 may be bleeding associated with pre-proliferative retinopathy.

However, in another example embodiment the region of interest 28 may be a location that is remote from the anomaly 26 detected in the first ophthalmic image. For example, if the anomaly 26 detected in the first ophthalmic image is indicative of widespread retinal bleeding, the like that would be indicative of pre-proliferative retinopathy (Stage 2 of diabetic retinopathy) then the region of interest 28 may be a region in the periphery of the patient's retina 21 and the predicted second anomaly 27 may be a retinal detachment. This would allow images to be captured of the peripheral region of the patient's retina 21 such that a clinician could acquire images of regions of the patient's retina that are at risk of retinal detachment. Retinal detachment is indicative of proliferative retinopathy (Stage 3 of diabetic retinopathy).

The size and position of the region of interest 28 may be determined based on characteristics of the detected anomaly and/or based on the predicted second anomaly 27. For example, if the anomaly 26 is indicative of a micro-aneurysm the region of interest 28 may be determined to be a region that encircles the anomaly 26 and the predicted second anomaly 27 may be an enlarged area of bleeding. The determined region of interest 28 may be positioned such that an OCT angiography image could be acquired of an enlarged area around the micro-aneurysm to assess the severity of retinal bleeding. However, if the anomaly 26 is indicative of widespread retinal bleeding the region of interest 28 may be a peripheral region of the retina 21 and the predicted second anomaly 27 may be a retinal detachment.

In another example, if the anomaly 26 is indicative of sub-retinal fluid the region of interest 28 may be a larger area that encompasses an area of between, for example, two and ten times the area of the detected anomaly 26. Determining the region of interest 28 may comprise determining a size and shape of the region of interest 28 based on characteristics of the detected anomaly 26 and/or based on a predicted second anomaly associated with a progressed stage of the multi-stage ocular disease associated with the detected pathology.

In Step 304 the second imaging modality to be used for imaging the determined region of interest 28 is determined based on the type of pathology or second anomaly 27 that is predicted to be present in the predicted progressed or second stage of the multi-stage ocular disease the detected anomaly 26 is indicative of. Determining the second imaging modality may comprise determining which of a plurality of imaging modalities will yield the highest quality ophthalmic image of the predicted second anomaly 27 such that the second ophthalmic image can be used for diagnosing a pathology that is predicted as being present in the determined region of interest 28. This may be performed using, for example, a machine learning algorithm trained on selecting an imaging modality based on a characteristic of an anomaly detected in the first ophthalmic image or from a look-up table that allows the second imaging modality to be selected based on a predicted progressed stage of the multi-stage ocular disease.

Returning to the above example, if the detected anomaly 26 is indicative of a micro-aneurysm which is synonymous with background retinopathy then the predicted progressed stage of the multi-stage ocular disease is pre-proliferative retinopathy and the predicted second anomaly 27 is retinal bleeding. Therefore, the second imaging modality may be selected based on the imaging modality that is best suited for assessing the severity of pre-proliferative retinopathy. For example, an OCT angiography imaging modality may be selected in this instance to visualise the severity of bleeding into the retina 21. However, if the anomaly was indicative of sub-retinal fluid, the second imaging modality may be selected based on the imaging modality that is best suited for assessing the severity and size of sub-retinal fluid to establish how progressed the spread of the sub-retinal fluid is. For example, an OCT imaging modality may be selected to obtain a cross-sectional image of the patient's retina such that the progression or spread of the sub-retinal fluid can be viewed in an OCT image.

Step 304 may further comprise selecting image acquisition characteristics or control parameters of the second imaging modality based on characteristics of the detected anomaly and/or based on the predicted pathology type associated with a progression of the anomaly detected in the first ophthalmic image. For example, the scan field, imaging depth, imaging wavelength, scan pattern and scan speed may be varied based on characteristics of the detected anomaly and/or based on the predicted pathology type associated with a progression of the anomaly detected in the first ophthalmic image to maximise the quality of the ophthalmic image captured by the second imaging modality.

Step 303 and Step 304 may further comprise receiving medical records indicative of the patient's medical history. Determining the region of interest in Step 303 may be based on a combination of the detected anomaly and the medical history of the patient to predict the second anomaly 27 that is associated with a progressed or second stage of the multi-stage ocular disease. Furthermore, selecting the second imaging modality in Step 304 for imaging the determined region of interest 28 may be performed based on a combination of the detected anomaly 26 and the medical history of the patient.

In Step 305 a second ophthalmic image is acquired of the determined region of interest 28 using a second imaging module operating in the selected second imaging modality selected in Step 304. The first and second imaging modules may be the same imaging module operating in different imaging modalities. The method may comprise displaying, storing and/or transmitting the first and/or second ophthalmic images.

The method may further comprise Step 306 to determine whether the predicted second anomaly 27 located in the determined region of interest 28 is captured within the second ophthalmic image. This is particularly beneficial when imaging anomalies that are indicative of sub-retinal disease. For example, if the anomaly is indicative of sub-retinal fluid, it may not be possible to assess the extent to which the sub-retinal fluid has spread until the second ophthalmic image has been captured and assessed.

Determining if the second anomaly 27 has been captured within the region of interest 28 in Step 306 may comprise determining if there are discontinuities at the border of the second ophthalmic image. If a discontinuity is detected at the border or edge of the second ophthalmic image frame then it me be inferred that the region of interest 28 determined in Step 303 was not large enough to encircle the second anomaly 27. For example, if the detected anomaly is indicative of sub-retinal fluid the second imaging modality selected to image the region of interest 28 may be an OCT imaging modality. Upon acquisition of the second ophthalmic image, which in this example would be an OCT image, the method may comprise determining if there are structural discontinuities at the frame boundaries of the second ophthalmic image thereby indicating that the region of interest 28, and thus OCT image field, should be larger in a given dimension to fully capture the second anomaly 27. In this case, the Step of 303 may be repeated to re-size the region of interest 28 based on the detection of a discontinuity at a boundary of the second ophthalmic image and the second ophthalmic image may be re-captured. This process may be repeated until no discontinuities are detected at the boundary of the second ophthalmic image thereby indicating that the second anomaly 28 has been fully captured within the second ophthalmic image.

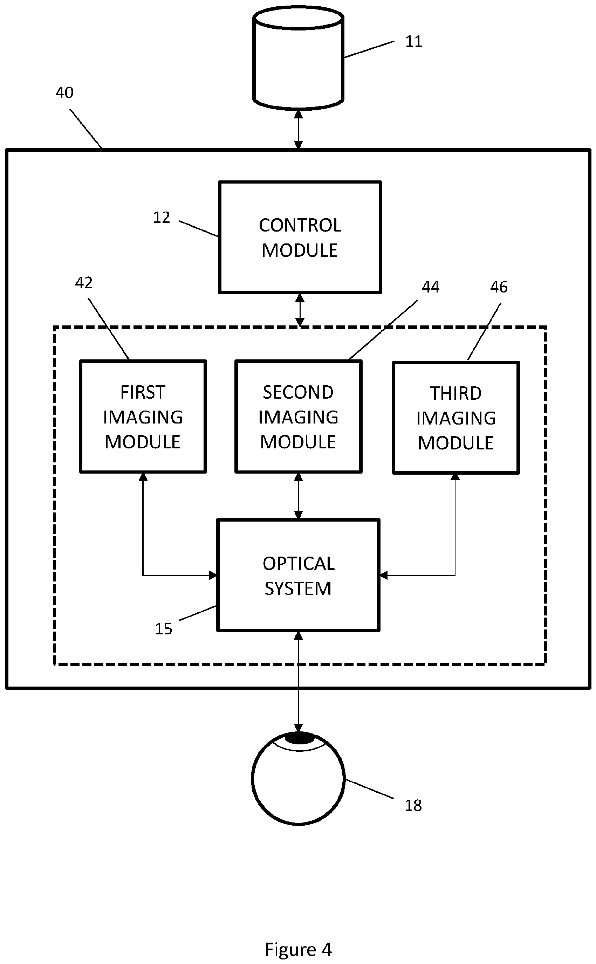

Figure 4 is a schematic illustration of a multi-modality ophthalmic imaging system 40 according to a second example embodiment herein. The multi-modality ophthalmic imaging system 40 comprises a first imaging module 42, a second imaging module 44, a third imaging module 44, an optical system 15 and a control module 12. Illustrated components and elements that are labelled with the same reference numerals as in the first example embodiment of Figure 1 are the same and will not be described again.

The multi-modality ophthalmic imaging system 40 comprises three imaging modules 42, 44, 46 each operable in one or more different imaging modalities. Each imaging module 42, 44, 46 is optically coupled to the optical system 15 and comprises a light source for emitting a light beam to the optical system for scanning across a target imaging area on the patient's eye 18. Furthermore, each imaging module may comprise one or more photodetectors for detecting light reflected back from the patient's eye 18 via the optical system 15. Each imaging module may further comprise a signal processing device arranged to generate ophthalmic images based on an output of the photodetector(s). Whilst the multi-modality ophthalmic imaging system 40 comprises three imaging modules 42, 44, 46 the skilled reader will understand that this is by way of example only and any number of imaging modules 42, 44, 46 may be implemented in the multi-modality ophthalmic imaging system 40.

As described above with reference to Figures 1 to 3, the first imaging module 42 is operable to acquire a first ophthalmic image. The first imaging module 42 may be a SLO imaging module (as shown in Figure 1) and operable in a fundus imaging modality to acquire a fundus image of the patient's eye.

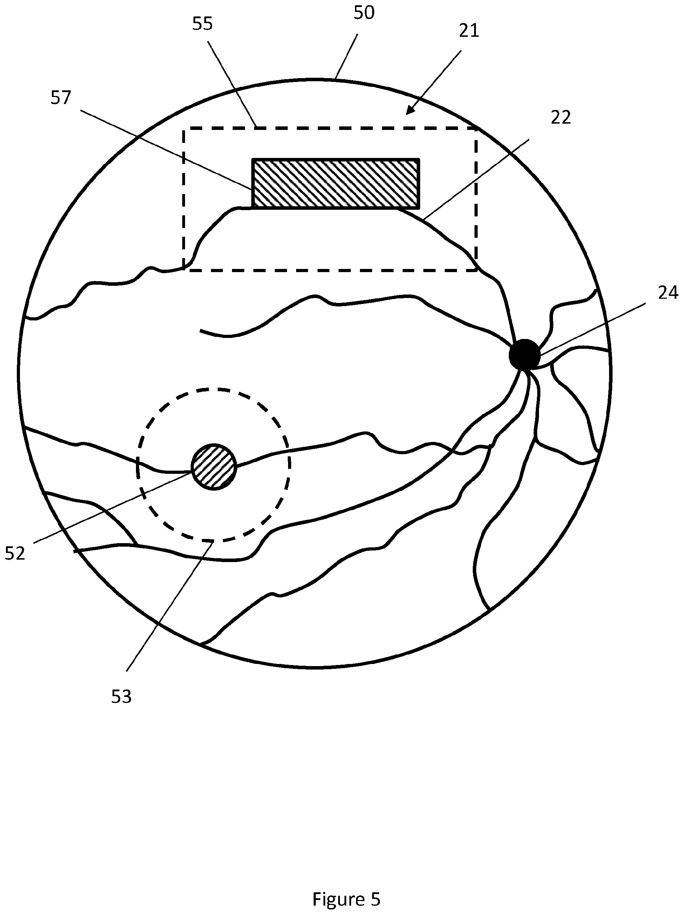

Figure 5 shows an example first ophthalmic image 50 captured by the first imaging module 42. As shown in Figure 5 the first ophthalmic image 50 comprises an anomaly 52 located on a branch of the retinal vasculature 22. The anomaly 52 shown in Figure 5 may be a micro-aneurysm or bulge that is leaking small amounts of blood. The anomaly 52 shown in Figure 5 may be an example of an anomaly 52 that is indicative of a multi-stage ocular disease such as diabetic retinopathy. As described above, the control module 12 is arranged to detect the presence of the anomaly 52 in the first ophthalmic image 50 and determine a stage of a multi-stage ocular disease the anomaly 52 is indicative of. In the example where the anomaly 52 is a micro-aneurysm the control module 12 may determine that the anomaly 52 is indicative of background retinopathy associated with the first stage in diabetic retinopathy.

Upon detection of the anomaly 52 in the first ophthalmic image 50 the control module 12 is arranged to determine a region of interest 28 that is predicted to contain a second anomaly 57 based on the detection of the anomaly 52 in the first ophthalmic image 50. In this example of an anomaly 52 indicative of a first stage of diabetic retinopathy, the control module 12 may determine that regions of interest associated with pathologies indicative of pre-proliferative retinopathy and proliferative retinopathy should be imaged to establish how progressed the diabetic retinopathy is in the patient. Pre-proliferative retinopathy and proliferative retinopathy are typically the second and third stage of progression in diabetic retinopathy patients respectively. Furthermore, the control module 12 may receive the patient's medical records from the database 11 that indicate the patient is diabetic thereby confirming that the anomaly 52 detected in the first ophthalmic image is likely to be indicative of diabetic retinopathy.

As shown in Figure 5, the control module 12 determines a first region of interest 53 that surrounds the anomaly 52 that should be imaged using a second imaging modality and a second region of interest 55 that is remote from the anomaly 52 and that is predicted to contain a second anomaly 57 that should be imaged by a third imaging modality.

The first region of interest 53 is a region that surrounds the anomaly 52. The first region of interest 53 is a region within the patient's eye 18 that is susceptible to the progression of the multi-stage ocular disease, in this example diabetic retinopathy. The anomaly 52 in this example is a bulge or micro-aneurysm that the control module 12 has determined is indicative of background retinopathy. To assess whether the diabetic retinopathy has progressed to the second stage of diabetic retinopathy, namely pre-proliferative retinopathy, the control module 12 has determined that a region surrounding the anomaly 52 to assess the extent of bleeding into the retina that is present as this is often the second stage in diabetic retinopathy patients. The control module 12 is arranged to determine a second imaging modality that should be selected to image the region of interest 53. In this example, the control module 12 may select an OCT angiography imaging modality to image the region of interest 53 to determine the extent of bleeding into the retina that is indicative of a second stage of diabetic retinopathy, namely pre-proliferative retinopathy.

The control module 12 may further identify a second region of interest 55 that is associated with a yet further progressed stage of the multi-stage ocular disease and that is predicted to contain a second anomaly 57. The second region of interest 55 may be a region within the patient's eye 18 that is remote from the anomaly 52 that is detected by the control module 12 in the first ophthalmic image. For example, in the instance where the control module 12 determines that the detected anomaly 52 is indicative of a first stage in diabetic retinopathy the second region of interest 55 may be a region that is susceptible to a retinal detachment and the predicted second anomaly 57 may be a retinal detachment. Retinal detachment can present in patient's that are suffering from proliferative retinopathy which is the third stage in diabetic retinopathy. The control module 12 may determine that a widefield or ultra-widefield infrared imaging modality should be selected to acquire a reflectance image of the second region of interest 55 located in the periphery of the patient's eye 18 to help a clinician accurately diagnose and assess the progression of retinal detachments by highlighting sub-retinal features that may not otherwise be visible in the first ophthalmic image.

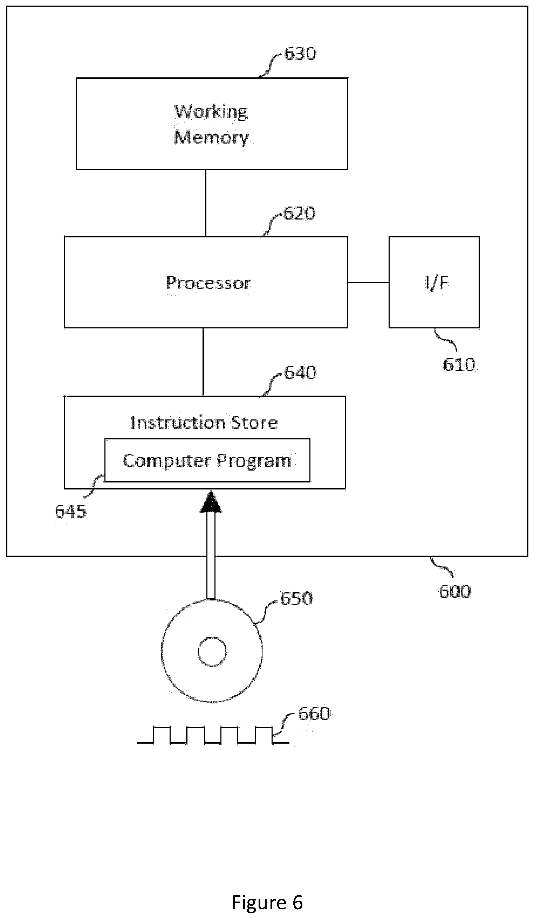

Figure 6 is a schematic illustration of a programmable signal processing hardware 600, configured to control imaging modules within a multi-modality ophthalmic imaging system 10. The programmable signal processing hardware 600 can perform at least part of the functionalities of the control module 12, and, in one example embodiment herein, at least part of the hardware 600 is included in the control module 12. The programmable signal processing apparatus 600 comprises a communication interface (I/F) 610, for receiving ophthalmic images acquired by the imaging modules and/or for outputting a determined imaging modality to operate a selected imaging module in the determined imaging modality. The communication interface (I/F) 610 can also receive medical records from the database 11. In one example embodiment herein, the communication interface (I/F) 610 can input/output any information obtained as part of the methods described herein.

The signal processing apparatus 600 further comprises a processor (e.g. a Central Processing Unit, CPU, and/or a Graphics Processing Unit, GPU) 620, a working memory 630 (e.g. a random access memory) and an instruction store 640 storing a computer program 645 comprising computer-readable instructions which, when executed by the processor 620, cause the processor 620 to perform various functions including those of the control module 12 in Figures 1 and 4, and/or the functions of the methods described herein. In one example embodiment herein, only the processor 620 is included in the control module 14, although in other examples one or more additional components of the hardware 600 also are included in the control module 12 as well.

The working memory 630 stores information used by the processor 620 during execution of the computer program 645. The instruction store 640 comprises, for example, a ROM (e.g. in the form of an electrically erasable programmable read-only memory (EEPROM) or flash memory) which is pre-loaded with the computer-readable instructions. Alternatively, the instruction store 640 comprises a RAM or similar type of memory, and the computer-readable instructions of the computer program 645 can be input thereto from a computer program product, such as a non-transitory, computer-readable storage medium 650 in the form of a CD-ROM, DVDROM, etc. or a computer-readable signal 660 carrying the computer-readable instructions. In any case, the computer program 645, when executed by the processor 620, causes the processor 620 to perform the methods described herein, including by example and without limitation, a method of imaging a patient's eye as described hereinabove.

In one example embodiment herein, the control module 12 of the example embodiments described above comprises the computer processor 620 and memory 640 storing the computer-readable instructions which, when executed by the computer processor 620, cause the computer processor 620 to perform the methods described herein, including by example and without limitation, a method of imaging a patient's eye using a multi-modality ophthalmic imaging system 10 as described herein.

In the foregoing description, example aspects are described with reference to several example embodiments. Accordingly, the specification should be regarded as illustrative, rather than restrictive. Similarly, the figures illustrated in the drawings, which highlight the functionality and advantages of the example embodiments, are presented for example purposes only. The architecture of the example embodiments is sufficiently flexible and configurable, such that it may be utilized in ways other than those shown in the accompanying figures.

Software embodiments of the examples presented herein may be provided as, a computer program, or software, such as one or more programs having instructions or sequences of instructions, included or stored in an article of manufacture such as a machine-accessible or machine-readable medium, an instruction store, or computer-readable storage device, each of which can be non-transitory, in one example embodiment. The program or instructions on the non-transitory machine-accessible medium, machine-readable medium, instruction store, or computer-readable storage device, may be used to program a computer system or other electronic device. The machine- or computer-readable medium, instruction store, and storage device may include, but are not limited to, floppy diskettes, optical disks, and magneto-optical disks or other types of media/machine-readable medium/instruction store/storage device suitable for storing or transmitting electronic instructions. The techniques described herein are not limited to any particular software configuration. They may find applicability in any computing or processing environment. The terms "computer-readable", "machine-accessible medium", "machine-readable medium", "instruction store", and "computer-readable storage device" used herein shall include any medium that is capable of storing, encoding, or transmitting instructions or a sequence of instructions for execution by the machine, computer, or computer processor and that causes the machine/computer/computer processor to perform any one of the methods described herein. Furthermore, it is common in the art to speak of software, in one form or another (e.g., program, procedure, process, application, module, unit, logic, and so on), as taking an action or causing a result. Such expressions are merely a shorthand way of stating that the execution of the software by a processing system causes the processor to perform an action to produce a result.

Some embodiments may also be implemented by the preparation of application-specific integrated circuits, field-programmable gate arrays, or by interconnecting an appropriate network of conventional component circuits.

Some embodiments include a computer program product. The computer program product may be a storage medium or media, instruction store(s), or storage device(s), having instructions stored thereon or therein which can be used to control, or cause, a computer or computer processor to perform any of the procedures of the example embodiments described herein. The storage medium/instruction store/storage device may include, by example and without limitation, an optical disc, a ROM, a RAM, an EPROM, an EEPROM, a DRAM, a VRAM, a flash memory, a flash card, a magnetic card, an optical card, nanosystems, a molecular memory integrated circuit, a RAID, remote data storage/archive/warehousing, and/or any other type of device suitable for storing instructions and/or data.

Stored on any one of the computer-readable medium or media, instruction store(s), or storage device(s), some implementations include software for controlling both the hardware of the system and for enabling the system or microprocessor to interact with a human user or other mechanism utilizing the results of the example embodiments described herein. Such software may include without limitation device drivers, operating systems, and user applications. Ultimately, such computer-readable media or storage device(s) further include software for performing example aspects of the invention, as described above.

Included in the programming and/or software of the system are software modules for implementing the procedures described herein. In some example embodiments herein, a module includes software, although in other example embodiments herein, a module includes hardware, or a combination of hardware and software.

While various example embodiments of the present invention have been described above, it should be understood that they have been presented by way of example, and not limitation. It will be apparent to persons skilled in the relevant art(s) that various changes in form and detail can be made therein. Thus, the present invention should not be limited by any of the above described example embodiments, but should be defined only in accordance with the following claims and their equivalents.

It is also to be understood that any procedures recited in the claims need not be performed in the order presented.

While this specification contains many specific embodiment details, these should not be construed as limitations on the scope of any inventions or of what may be claimed, but rather as descriptions of features specific to particular embodiments described herein. Certain features that are described in this specification in the context of separate embodiments can also be implemented in combination in a single embodiment. Conversely, various features that are described in the context of a single embodiment can also be implemented in multiple embodiments separately or in any suitable sub-combination. Moreover, although features may be described above as acting in certain combinations and even initially claimed as such, one or more features from a claimed combination can in some cases be excised from the combination, and the claimed combination may be directed to a sub-combination or variation of a sub-combination.

In certain circumstances, multitasking and parallel processing may be advantageous. Moreover, the separation of various components in the embodiments described above should not be understood as requiring such separation in all embodiments, and it should be understood that the described program components and systems can generally be integrated together in a single software product or packaged into multiple software products.

## Claims

1. A multi-modal ophthalmic imaging system (10, 40) for acquiring ophthalmic images of a patient's eye (18), the multi-modal ophthalmic imaging system (10, 40) comprising:
a first imaging module (14, 42), operable in a first imaging modality, for acquiring a first ophthalmic image (20, 50) of the patient's eye (18);
one or more further imaging modules (16, 44, 46), each operable in a respective imaging modality, wherein each imaging modality of the one or more further imaging modules (16, 44, 46) is different to the first imaging modality; and
a control module (12) arranged to:
detect an anomaly (26, 52) in the first ophthalmic image (20, 50) acquired by the first ophthalmic imaging module (14, 42);
determine a region of interest (28, 53, 55) in the patient's eye based on the detected anomaly in the first ophthalmic image, wherein the determined region of interest (28, 53, 55) is a region within the patient's eye (18) that is predicted to contain a second anomaly (27, 57);
determine, based on the second anomaly (26, 52), a second imaging modality for imaging the determined region of interest (28, 53, 55), the second imaging modality being different from the first imaging modality;
select, from the one or more further imaging modules (16, 44, 46), a second imaging module (16, 44, 46) which is operable in the determined second imaging modality; and
control the selected second imaging module (16, 44, 46) to acquire a second ophthalmic image of the determined region of interest (28, 53, 55).

2. An ophthalmic imaging system as claimed in Claim 1, wherein the anomaly (26, 52) detected in the first ophthalmic image is indicative of a first stage of a multi-stage ocular disease and wherein the second anomaly (27, 57) is indicative of a second stage of the multi-stage ocular disease.

3. An ophthalmic imaging system as claimed in Claim 1 or Claim 2, wherein the first imaging module (14, 42) comprises a scanning laser ophthalmoscope (SLO), and the first ophthalmic image is a fundus image of the patient's eye (18).

4. An ophthalmic imaging system as claimed in any preceding claim, wherein the control module (12) is further arranged to determine, based on the second anomaly (27, 57), one or more image acquisition parameters of the second imaging module (16, 44) for controlling the second imaging module (16, 44) to acquire the second ophthalmic image of the determined region of interest (28, 53).

5. An ophthalmic imaging system as claimed in any preceding claim, wherein the control module (12) is communicatively coupled to a database (11) comprising a medical record indicative of a medical history of the patient, and wherein the control module (12) is arranged to retrieve the medical record of the patient from the database (11) and determine the region of interest (28, 53) based on both the detected anomaly (26) and the medical history of the patient.

6. An ophthalmic imaging system as claimed in any preceding claim, wherein the control module (12) is configured to detect a discontinuity at a boundary of the second ophthalmic image, wherein the discontinuity at the boundary is indicative of cropping of the second anomaly (27, 57) located within the second ophthalmic image.

7. An ophthalmic imaging system as claimed in Claim 6, wherein the control module (12) is arranged, in response to detecting the discontinuity at the boundary of the second image, to
increase a size of the determined region of interest to generate an enlarged region of interest; and
control the second imaging module to acquire an ophthalmic image of the enlarged region of interest.

8. An ophthalmic imaging system as claimed in any preceding claim, wherein the control module is arranged to:
determine a second region of interest based on the detected anomaly wherein the second region of interest is a region that contains the detected anomaly;
determine, based on the detected anomaly, a third imaging modality for imaging the determined second region of interest, the third imaging modality being different from the first imaging modality;
select, from the one or more further imaging modules, a third imaging module which is operable in the determined third imaging modality; and
control the selected third imaging module to acquire a third ophthalmic image of the determined second region of interest.

9. A method of imaging a patient's eye (18), the method comprising:
acquiring a first ophthalmic image (20, 50) of the patient's eye (18) using a first imaging module (14, 42) operating in a first imaging modality;
detecting an anomaly (26, 52) in the first ophthalmic image (20, 50) acquired by the first ophthalmic imaging module (14, 42);
determining a region of interest (28) in the patient's eye (18) based on the detected anomaly (26) in the first ophthalmic image, wherein the determined region of interest (28) is a region within the patient's eye (18) that is predicted to contain a second anomaly (27, 57);
determining, based on the second anomaly (20, 50), a second imaging modality for imaging the determined region of interest (27, 57), the second imaging modality being different from the first imaging modality; and
acquiring a second ophthalmic image of the determined region of interest (27, 57) using a second imaging module (16, 44) operating in the determined second imaging modality.

10. A method as claimed in Claim 9, wherein the anomaly (26) detected in the first ophthalmic image is indicative of a first stage of a multi-stage ocular disease and wherein the second anomaly (27) is indicative of a second stage of the multi-stage ocular disease.

11. A method as claimed in Claim 9 or Claim 10, further comprising determining if the second anomaly is captured within the second ophthalmic image by detecting a discontinuity at a boundary of the second ophthalmic image, wherein detecting the presence of a discontinuity at the boundary is indicative of cropping of the second anomaly within the second ophthalmic image.

12. A method as claimed in any one of Claims 9 to 11, comprising, in response to detecting the presence of the discontinuity at the boundary of the second ophthalmic image, increasing a size of the determined region of interest to generate an enlarged region of interest and acquiring a further ophthalmic image of the enlarged region of interest.

13. A method as claimed in any one of Claims 9 to 12, comprising receiving medical records of the patient containing a medical history of the patient, wherein determining the region of interest is based on a combination of the detected anomaly in the first ophthalmic image and the received medical history of the patient.

14. A method as claimed in any one of Claims 9 to 13, further comprising determining, based on the second anomaly, one or more image acquisition parameters of the second imaging module and operating the second imaging module in the determined image acquisition parameters to acquire the second ophthalmic image.

15. A method as claimed in any one of Claims 9 to 14, further comprising:
determining a second region of interest based on the detected anomaly, wherein the second region of interest is a region that contains the detected anomaly;
determining, based on the detected anomaly, a third imaging modality for imaging the determined second region of interest, the third imaging modality being different from the first imaging modality;
selecting, from the one or more further imaging modules, a third imaging module which is operable in the determined third imaging modality; and
controlling the selected third imaging module to acquire a third ophthalmic image of the determined second region of interest.
